# EUROPÄISCHE PATENTANMELDUNG

(11) **EP 2 314 248 A1**
(43) Veröffentlichungstag der Anmeldung: **27.04.2011**
(21) Anmeldenummer: 09401023.8
(22) Anmeldetag: 20.10.2009
(51) Int. Cl.: A61B 19/00

(54) **Spülvorrichtung für Saug- und Spülkanülen**

(71) Anmelder: Miele & Cie. KG, 33332 Gütersloh (DE)
(72) Erfinder: Michiels, Winfried, 34414 Warburg (DE)

(57) **Zusammenfassung**

Die Erfindung betrifft eine Spülvorrichtung für Saug- und Spülkanülen (9), zum Anschluss an die Spülflüssigkeitszuleitung eines Reinigungs- und Desinfektionsautomaten, wobei die Kanülen (9) ein Anschluss- und Halterungsteil (6, 61, 62), ein Kanülenteil (7, 71, 72), und gegebenenfalls ein Stell- oder Ventilelement (8) umfassen. Damit eine verbesserte Reinigung der Kanülen (7) möglich ist, wird vorgeschlagen, dass die Spülvorrichtung aus einem mindestens zweiteiligen Aufnahmegehäuse (20) besteht, welches an die Spülflüssigkeitszuleitung ankoppelbar ist und welches eine Aufnahme der Kanüle (9) in der Art ermöglicht, dass das Anschluss- und Halterungsteil (6) und das gegebenenfalls vorhandene Stell- oder Ventilelement (8) im Aufnahmegehäuse (20) angeordnet sind und dass das Kanülenteil (7, 71, 72) aus einer Öffnung (27) im Aufnahmegehäuse (20) ragt.

## Beschreibung

Die Erfindung betrifft eine Spülvorrichtung für Saug- und Spülkanülen zum Anschluss an die Spülflüssigkeitszuleitung eines Reinigungs- und Desinfektionsautomaten, wobei die Kanülen ein Anschluss- und Halterungsteil, ein Kanülenteil und gegebenenfalls ein Stell- oder Ventilelement umfassen.

Die Reinigung von Saug- und Spülkanülen in gewerblichen Reinigungs- und Desinfektionsautomaten ist aufgrund der großen Variationsbreite bei der Form und Funktionsweise solcher Kanülen und wegen fehlender Anschlussstandards sehr schwierig. Zum einen erfolgt sowohl im Bereich der Spülflüssigkeitszuleitung in die Kanüle als auch durch Öffnungen in Stell- und Ventilelementen aufgrund von Undichtigkeiten ein Druckverlust, welcher eine zufriedenstellende Reinigung nicht zulässt. Zum anderen ist eine Halterung der Kanülen während des Spülprozesses notwendig, bei der häufig eine große Kontaktfläche zwischen Halterungselementen und der Kanüle selbst gegeben ist. An diesen Stellen entstehen Bereiche, in denen Schmutz verdeckt wird und demzufolge nicht gereinigt werden kann. Außerdem sammelt sich aufgrund des Kapillareffekts kontaminierte Spülflüssigkeit und verschlechtert das Reinigungsergebnis.

Der Erfindung stellt sich somit das Problem eine Spülvorrichtung für Saug- und Spülkanülen der eingangs genannten Art zu offenbaren mit der eine verbesserte Reinigung der Kanülen möglich ist.

Erfindungsgemäß wird dieses Problem durch eine Spülvorrichtung mit den Merkmalen des Patentanspruchs 1 gelöst. Vorteilhafte Ausgestaltungen und Weiterbildungen der Erfindung ergeben sich aus den nachfolgenden Unteransprüchen.

Die mit der Erfindung erreichbaren Vorteile bestehen einerseits darin, dass durch eine flüssigkeitsdichte Ankopplung des Aufnahmegehäuses an die Spülflüssigkeitszuleitung des Reinigungs- und Desinfektionsautomaten Druckverluste vermieden werden und demzufolge die Kanülen auch im Inneren gut durchgespült werden. Andererseits wird die Kanüle mit nur punktuellem Kontakt in dem Aufnahmegehäuse gehaltert und dieses zudem von Trocknungsluft durchströmt, so dass sowohl eine Schmutzabdeckung als auch eine Ansammlung von Restflüssigkeit verhindert wird.

In einer vorteilhaften Ausführungsform ist ein erstes Teil des Aufnahmegehäuses mit einem Anschluss für die Spülflüssigkeitszuleitung und ein zweites Teil mit der Öffnung ausgestattet. Hierdurch wird das Handling bei Unterbringen einer Kanüle in dem Aufnahmegehäuse vereinfacht. Es ist dann besonders einfach, wenn das erste und zweite Teil mittels eines Bajonettverschlusses verbindbar sind. Alternativ können das erste und zweite Teil mittels eines Schraubverschlusses verbindbar sein.

Ein besonderer Vorteil ergibt sich, wenn der Querschnitt der Öffnung verstellbar ist. Hierdurch können Druckverluste im Bereich der Durchführung des Kanülenteils durch das Aufnahmegehäuse vermieden werden. Eine solche Querschnittsveränderung ist in einfacher Weise dadurch zu erreichen, dass in einer Deckplatte des zweiten Teils eine erste Öffnung mit großem Querschnitt angeordnet ist und dass vor der Deckplatte eine Verstellplatte mit zweiten Öffnungen mit verschiedenen Querschnitten angeordnet ist, wobei von den zweiten Öffnungen jeweils eine vor die erste Öffnung bringbar ist.

Eine ausreichende Prozesssicherheit wird dadurch erreicht, dass das Aufnahmegehäuse aus Metall, vorzugsweise aus Edelstahl gefertigt ist. Ein Ausführungsbeispiel der Erfindung ist in den Zeichnungen rein schematisch dargestellt und wird nachfolgend näher beschrieben. Es zeigt
- Figur 1: einen mit Reinigungsgut beladenen Einsatzwagen für einen gewerblichen Reinigungs- und Desinfektionsautomaten,
- Figur 2: die Spülflüssigkeitszuleitung eines Einsatzwagens mit aufgesteckter Veres-Kanüle, rechts äußerer Teil, links innerer Teil,
- Figur 3: einen Querschnitt durch ein erfindungsgemäßes Aufnahmegehäuse mit eingelegter Kanüle,
- Figur 4: ein erfindungsgemäßes Aufnahmegehäuse in perspektivischer Darstellung.

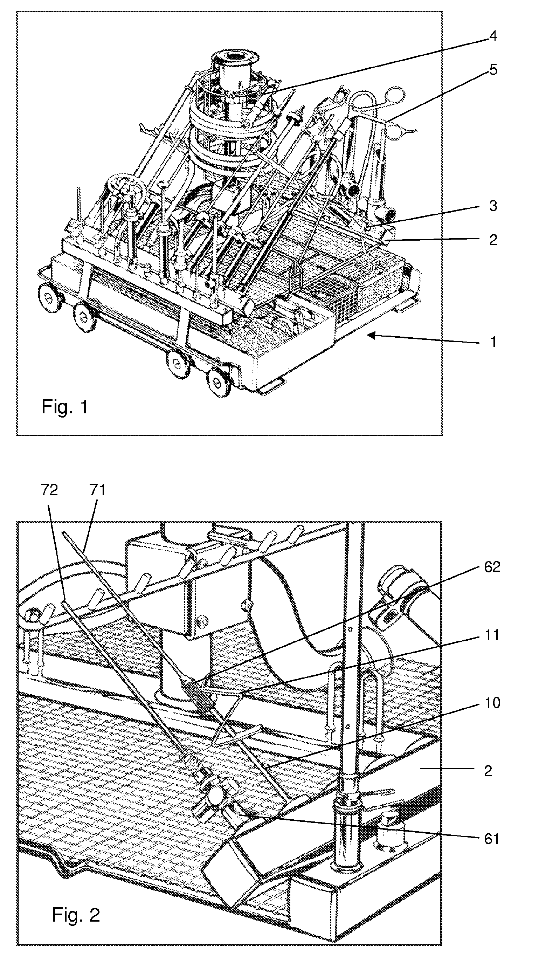

Die Figur 1 zeigt einen an sich bekannten Einsatzwagen 1 für einen gewerblichen Reinigungs- und Desinfektionsautomaten. Letzterer ist beispielsweise aus der DE 34 13 386 A1 bekannt und wird deshalb hier weder näher beschrieben noch in den Zeichnungen dargestellt. Zur Erklärung der Funktion der erfindungsgemäßen Spülvorrichtung ist es lediglich wichtig, dass der Einsatzwagen 1 in den Spülraum des Reinigungs- und Desinfektionsautomaten eingeschoben und dabei an eine Spülflüssigkeitszuleitung angekoppelt wird. Dadurch kann die Spülflüssigkeit am Einsatzwagen in einen Verteiler 2 eintreten, welcher wiederum mit verschiedenen Anschlüssen 3 für durchzuspülendes Spülgut ausgestattet ist. In der Figur 1 sind beispielhaft Schläuche 4 und verschiedene MIC-Instrumente 5 als Spülgut gezeigt. Unter anderem dient ein solcher Einsatzwagen 1 auch zur Reinigung von Saug- und Spülkanülen.

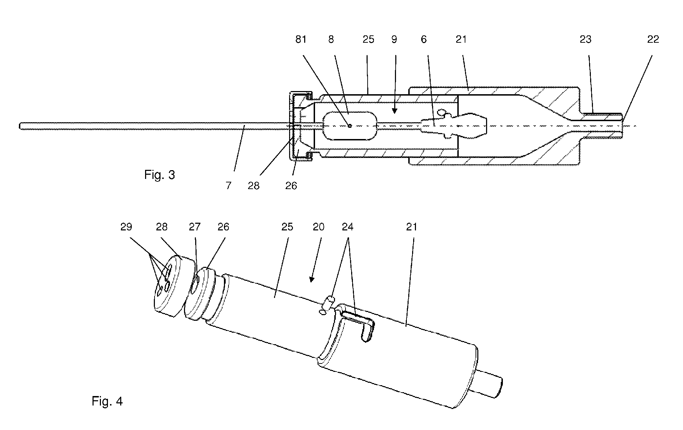

Solche Kanülen können, wie in Figur 2 am Beispiel einer Veres-Kanüle gezeigt, mehrteilig aufgebaut sein. Es gibt aber auch einfache einteilige Kanülen 9, wie in Figur 3 dargestellt.

Wesentliche Bestandteile einer Kanüle 9 sind ein Anschluss- und Halterungsteil 6, über welches die Kanülen 9 mit einer externen Saugeinrichtung oder Gas- und/oder Flüssigkeitszufuhr verbunden sind, und das eigentliche Kanülenteil 7 in Form einer Hohlnadel. Die in Figur 2 gezeigte Veres-Kanüle umfasst zwei ineinandergesteckte Kanülenteile 71 und 72, die in Figur 3 gezeigte Kanüle 9 besitzt zusätzlich einen Saugunterbrecher 8, welcher im Wesentlichen als vom Daumen des Operateurs verschließbare Öffnung 81 ausgebildet ist.

Aus der Figur 2 ist außerdem zu erkennen, wie beispielsweise Veres-Kanülen nach dem Stand der Technik gereinigt werden. Dabei ist das innere Kanülenteil 72 mittels eines Luer-Lock-Anschlusses 61 mit dem Verteiler 2 des Einsatzwagens 1 gekoppelt, welcher die Spülflüssigkeitszuleitung aus dem Reinigungs- und Desinfektionsautomaten fortsetzt. Durch den flüssigkeitsdichten Luer-Lock-Anschluss 61 durchfließt ausreichend Spülflüssigkeit das innere Kanülenteil 72 und reinigt es zufriedenstellend. Die Reinigung des äußeren Kanülenteils 71 der Veres-Kanüle ist schwieriger. Da dort ein genormter Anschluss fehlt, kann das Anschluss- und Halterungsteil 62 nur auf ein Spülrohr 10 gesteckt und mittels einer Federklemme 11 gehalten werden. Durch den undichten Übergang und den Rückstau, der durch das dünne Kanülenteil 71 erzeugt wird, gelangt ein großer Anteil an Spülflüssigkeit nicht in das Kanülenteil 71, sondern fließt schon im Bereich des Anschluss- und Halterungsteils 62 ab. Es entsteht ein Druckverlust und dadurch ist das Reinigungsergebnis nicht immer zufriedenstellend. Außerdem berührt das Anschluss- und Halterungsteil 62 im Inneren auf einer großen Fläche das Spülrohr 10, was zu einer Schmutzabdeckung und/oder zu einer Ansammlung von Restflüssigkeit führt.

Hier hilft das erfindungsgemäß ausgebildete Aufnahmegehäuse 20. Dieses umfasst, wie in den Figuren 3 und 4 ersichtlich, ein erstes Teil 21 mit einem Anschluss 22 für die Spülflüssigkeitszuleitung. Im gezeigten Ausführungsbeispiel ist dies ein Gewinde 23, welches kompatibel mit dem Standard-Gewinden im Verteiler 2 (bspw. Für den gezeigten Luer-Lock-Anschluss) ist und somit einfach in den Verteiler 2 geschraubt werden kann. Mittels eines Bajonett-Verschlusses 24 (siehe Figur 4) wird ein zweites Teil 25 mit dem ersten 21 verbunden, nachdem die Kanüle 9 eingelegt ist. Das zweite Teil 25 besitzt in seiner Deckplatte 26 eine erste Öffnung 27, durch die das Kanülenteil 7 durchgeführt werden kann. Um Variationsmöglichkeiten für unterschiedliche Kanülenteil-Querschnitte zu haben, ist die erste Öffnung 27 exzentrisch in der Deckplatte 26 angeordnet und davor eine Verstellplatte 28 mit zweiten Öffnungen 29 mit verschiedenen Querschnitt vorgesehen. Durch Drehen der Verstellplatte 28 kann die jeweils passende zweite Öffnung 29 vor die erste Öffnung 27 gebracht werden, so dass das Kanülenteil 7 wenigstens annähernd flüssigkeitsdicht umfasst ist. Die durch Undichtigkeiten an der Außenseite des Kanülenteils 7 entlang strömende Spülflüssigkeit sorgt für eine Reinigung dieses Bereichs. Ein langlochförmiger Querschnitt der zweiten Öffnungen 29 sorgt dafür, dass auch abgewinkelte Kanülenteile 7 aufgenommen werden können. Aus der Figur 3 ist erkennbar, dass nach dem Verbinden des ersten 21 und zweiten Teils 25, das Anschluss- und Halterungsteil 6 und der Saugunterbrecher 8 im Aufnahmegehäuse 20 angeordnet sind.

Das Aufnahmegehäuse 20 ist vorzugsweise aus Edelstahl gefertigt, um eine ausreichende Verschleißfestigkeit bei zahlreichen Reinigungsvorgängen zu gewährleisten.

## Patentansprüche

**1.** Spülvorrichtung für Saug- und Spülkanülen (9), zum Anschluss an die Spülflüssigkeitszuleitung eines Reinigungs- und Desinfektionsautomaten, wobei die Kanülen (9) ein Anschluss- und Halterungsteil (6, 61, 62), ein Kanülenteil (7, 71, 72), und gegebenenfalls ein Stell- oder Ventilelement (8) umfassen,
**dadurch gekennzeichnet,**
**dass** die Spülvorrichtung aus einem mindestens zweiteiligen Aufnahmegehäuse (20) besteht, welches an die Spülflüssigkeitszuleitung ankoppelbar ist und welches eine Aufnahme der Kanüle (9) in der Art ermöglicht, dass das Anschluss- und Halterungsteil (6) und das gegebenenfalls vorhandene Stell- oder Ventilelement (8) im Aufnahmegehäuse (20) angeordnet sind und dass das Kanülenteil (7, 71, 72) aus einer Öffnung (27) im Aufnahmegehäuse (20) ragt.

**2.** Spülvorrichtung nach Anspruch 1,
**dadurch gekennzeichnet,**
**dass** ein erstes Teil (21) des Aufnahmegehäuses (20) mit einem Anschluss (22) für die Spülflüssigkeitszuleitung und ein zweites Teil (25) mit der Öffnung (27) ausgestattet ist.

**3.** Spülvorrichtung nach Anspruch 2,
**dadurch gekennzeichnet,**
**dass** das erste (21) und zweite Teil (25) mittels eines Bajonettverschlusses (24) verbindbar sind.

**4.** Spülvorrichtung nach Anspruch 2,
**dadurch gekennzeichnet,**
**dass** das erste (21) und zweite Teil (25) mittels eines Schraubverschlusses verbindbar sind.

**5.** Spülvorrichtung nach mindestens einem der vorstehenden Ansprüche,
**dadurch gekennzeichnet,**
**dass** der Querschnitt der Öffnung (27) verstellbar ist.

**6.** Spülvorrichtung nach Anspruch 5,
**dadurch gekennzeichnet,**
**dass** in einer Deckplatte (26) des zweiten Teils (25) eine erste Öffnung (27) mit großen Querschnitt angeordnet ist, und dass vor der Deckplatte (26) eine Verstellplatte (28) mit zweiten Öffnungen (29) mit verschiedenen Querschnitten angeordnet ist, wobei von den zweiten Öffnungen (29) jeweils eine vor die erste Öffnung (27) bringbar ist.

**7.** Spülvorrichtung nach mindestens einem der vorstehenden Ansprüche,
**dadurch gekennzeichnet,**
**dass** das Aufnahmegehäuse (20) aus Metall, vorzugsweise aus Edelstahl gefertigt ist.
